# EUROPEAN PATENT APPLICATION

(11) **EP 1 767 534 A1**
(43) Date of publication of application: **28.03.2007**
(21) Application number: 04792627.4
(22) Date of filing: 13.10.2004
(51) Int. Cl.: C07D 403/10, A61K 31/4184, A61P 3/06, A61P 9/12, A61P 19/06

(54) **DRUG FOR TREATING OR PREVENTING DUPLICATION OF HYPERTENSION WITH SERUM HYPERURICEMIA AND/OR HYPERCHOLESTEROLEMIA**

(30) Priority: 23.06.2004 JP 2004184906
(71) Applicant: Kotobuki Pharmaceutical Co., Ltd., Hanishina-gun, Nagano 389-0601 (JP)
(72) Inventor: TOMIYAMA, Akira, Hanishina-gun, Nagano 389-0602 (JP); TOMIYAMA, Hiroshi, Hanishina-gun, Nagano 389-0601 (JP); MIYAMOTO, Hidetoshi, Chikuma-shi, Nagano 387-0024 (JP); HAYASHI, Keiichiro, Ueda-shi, Nagano 386-0011 (JP); MOCHIZUKI, Seiichiro, Ueda-shi, Nagano 386-1102 (JP)
(74) Representative: Albrecht, Thomas
(86) International application number: PCT/JP2004/015461
(87) International publication number: WO 2006/001090

(57) **Abstract**

The purpose in this invention is to providing drugs for treating or preventing duplication of hypertension with serum hyperuricemia and/or hypercholesterolemia that is much frequency among the duplication onset in geriatric diseases. This invention is related to drugs for treating or preventing duplication of hypertension with serum hyperuricemia and/or hypercholesterolemia of which active principles are 2-propyl-3- {[2'-(1H-tetrazole -5-yl) biphenyl -4-yl]methyl}-5, 6, 7, 8-tetrahydrocyclohepta imidazole -4-(3H) - one and that prodrug or that salt. Pratosartan is able to use in combination with one or more than two diuretics chosen from sulfonamide-, phenoxyacetic acid- and thiazide-type diuretics, triamterene, amiloride, spironolactone, potassium canrenoate and traxanox sodium. Also, pratosartan is able to use in combination with one or more than two hypolipidemic drugs chosen from statins, fibrates, cholesterol absorption inhibitors, cholesterol sequestrants and cholesterol excretion enhancers.

## Description

### TECHNICAL FIELD

This invention is related to drugs for treating or preventing duplication of hypertension with hyperuricemia and/or hypercholesterolemia.

### BACKGROUND TECHNOLOGY

Recently, various studies for prevention and therapy of geriatric diseases are performed. Risk factors of geriatric diseases are hypertension, hypercholesterolemia, diabetes mellitus, obesity and hyperuricemia. And, the mortality markedly increases when these risk factors overlap more than two. Also, it is known that among this overlap of the risk factor, the overlap of hypertension and hyperuricemia, of hypertension and hypercholesterolemia are much frequency.

Conventionally, angiotensin II receptor blockers are widely used for therapy of hypertension. And, the inhibitory action on atherosclerosis development of angiotensin II receptor blockers is shown in animal experiments as secondary or indirect action of hypotensive effect. However, there is no report that angiotensin II receptor blocker decreases plasma cholesterol level or LDL level. It is known that 2-propyl-3-{[2'-(1H-tetrazole-5-yl)biphenyl-4-yl]methyl}-5, 6, 7, 8-tetrahydrocyclohepta imidazole -4-(3H) - one (General name : pratosartan) in this invention has angiotensin II receptor blocking action and is effective for therapy of hypertension and cardiac failure (JPB 707390). It is known for a long time that most diuretics, which are generally used same as angiotensin II receptor blockers in clinical situation, itself increase uric acid levels, and it is reported that sulfonamide- and thiazide-type diuretics increase serum uric acid levels [Joel G. Hardmann et al., The Pharmacological Basis of Therapeutics, 10th edition, McGraw-Hill (USA), 2001, page 757-787] . Also, it is reported that some of angiotensin II receptor blockers increase uric acid levels or have no uric acid excretion action (Saitoh, M. et al., "Uricosuric Effects of Angiotensin II Receptor Antagonist in the Patients with Normal Renal Function", Japanese Journal of Clinical Pharmacology and Therapeutics, 2003, 34(2), 37-42. "Journal of Hypertensions", 2001, 19(10), 1855-1860). Nevertheless, conventional co-administration of angiotensin II blocker and diuretics is an important therapeutic method because of certain hypotensive action (International publication No. 89/6233 pamphlet, JPA H3(1991)-27362). In the guideline for hypertension therapy, in regard to uric acid level of hypertension, it is desirable to choose the hypotensive drugs that are not increase uric acid level at least. (Guideline Subcommittee of the Japanese Society of Hypertension, JSH2000 Hypertension Guidelines for General Practitioners, 1st edition, The Japanese Society of Hypertension, June 30, 2000, page 55-58). In the view of this increase of uric acid, there are some problems about conventional co-administration of angiotensin II blocker and diuretics. Under these circumstances, angiotensin II blockers, which are able to decrease uric acid level, are expected, and the dosage of those alone or a combination drug are also expected.

Also, it is known that high dose of thiazide- and loop-type diuretics increase serum cholesterol level, triglyceride and LDL-cholesterol (Guideline Subcommittee of the Japanese Society of Hypertension, JSH2000 Hypertension Guidelines for General Practitioners, 1st edition, The Japanese Society of Hypertension, June 30, 2000, page 55-58). In hypercholesterolemia, sufficient hypocholesterolemic effect is not obtained when one drug is used occasionally, so co-administration by multiple hypocholesterolemic drugs is performed in clinical situation generally.

In this invention, the purpose is to providing drugs for treating or preventing duplication of hypertension with serum hyperuricemia and/or hypercholesterolemia that is much frequency among the duplication onset in geriatric diseases.

### DISCLOSURE OF INVENTION

Inventors of this invention newly discovered that 2-propyl-3- {[2'-(1H-tetrazole -5-yl) biphenyl -4-yl]methyl}-5, 6, 7, 8-tetrahydrocyclohepta imidazole -4-(3H) - one (General name : pratosartan), which is one of the angiotensin II blockers, have hypouricosuric action and hypolipidemic action (hypocholesterolemic action and LDL lowering action) other than hypotensive action by animal studies and clinical trials, and completed this invention.

In other words, this invention is related to drugs for treating or preventing duplication of hypertension with serum hyperuricemia and/or hypercholesterolemia of which active principles are 2-propyl-3- {[2'-(1H-tetrazole -5-yl) biphenyl -4-yl]methyl}-5, 6, 7, 8-tetrahydrocyclohepta imidazole -4-(3H) - one and that prodrug or that salt.

Also, this invention is related to drugs for treating or preventing duplication of hypertension with serum hyperuricemia and/or hypercholesterolemia of which active principles are 2-propyl-3- {[2'-(1H-tetrazole -5-yl) biphenyl -4-yl]methyl }-5, 6, 7, 8-tetrahydrocyclohepta imidazole -4-(3H) - one and that prodrug or that salt and diuretics. As diuretics of the above, one or more than two diuretics are preferable chosen from sulfonamide-, phenoxyacetic acid- and thiazide-type diuretics, triamterene, amiloride, spironolactone, potassium canrenoate and traxanox sodium. Also, as diuretics of the above, thiazide-type diuretics are preferable chosen from the group consist of hydrochlorothiazide, methyclothiazide, benzylhydrochlorothiazide, trichloromethiazide, cyclopenthiazide, polythiazide, ethiazide, cyclothiazide, bendroflumethiazide and hydroflumethiazide.

Also, this invention is related to drugs for treating or preventing duplication of hypertension with serum hyperuricemia and/or hypercholesterolemia, of which active principles are 2-propyl-3- {[2'-(1H-tetrazole -5-yl) biphenyl -4-yl]methyl}-5, 6, 7, 8-tetrahydrocyclohepta imidazole -4-(3H) - one and that prodrug or that salt and hypolipidemic drugs. As hypolipidemic drugs of the above, one or more than two diuretics are preferable chosen from the group consist of statins, fibrates, cholesterol sequestrants, cholesterol absorption inhibitors and cholesterol excretion enhancers.

### BRIEF DESCRIPTION OF DRAWING

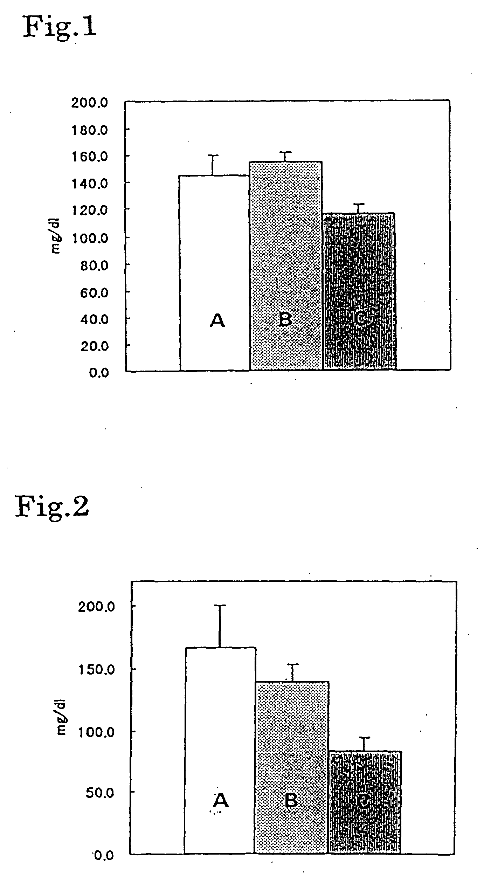

Figure 1 is a graph showing the result of serum total cholesterol measured, and figure 2 is a graph showing the result of LDL-cholesterol measured.

### BEST MODE FOR CARRYING OUT THE INVENTION

Pratosartan alone or mixed with appropriate pharmaceutical acceptable excipients or dilutions, for example oral dosage such as tablet, capsule, granule, powder or syrup, or non-oral dosage such as suppository. Though the dose is varied by condition, age and others, in oral dosage, the dose of pratosartan varies from 1 mg of lower limit to 1000 mg of upper limit. The dosage number is from one to five.

Pratosartan has plasma uric acid level decreasing action, and is useful as plasma uric acid excretion enhancer and uricosuric drug. Also, because of the hypotensive action of pratosartan, it is expected to ameliorate hypertension with hypouricemia, and is useful for prevention or therapy of hyperuricemia with hypertension. Furthermore, pratosartan has hypocholesterolrmic action and is useful as hypolipidemic drug of plasma cholesterol. Also, because of the hypotensive action of pratosartan, it is expected to ameliorate hypertension with hypolipidemia or atherosclerosis, and is useful for prevention or therapy of hypolipidemia or atherosclerosis with hypertension.

Also, pratosartan is able to use in combination with diuretics. Though diuretics, especially sulfonamide- and thiazide-type diuretics, have hypotensive action, those have adverse effect of increasing plasma uric acid level. On the other hand, many of conventional angiotensin II receptor blockers increase plasma uric acid level. Therefore, it is difficult to administer diuretics with conventional angiotensin II receptor blockers. However, pratosartan has an advantage of decreasing plasma uric acid level, so pratosartan co-administered with diuretics show hypotensive action of diuretics with inhibition of increasing plasma uric acid level. And, because pratosartan itself show hypotensive action, synergistic hypotensive actions are obtained by co-administration with diuretics. Co-administration of pratosartan with diuretics are expected as preventive or therapeutic drug for hypertension, hypertension with hyperuricemia and cardiovascular diseases. Also, though it is known that high dose of thiazide- and loop-type diuretics increase serum total cholesterol, triglyceride and LDL cholesterol (Guideline Subcommittee of the Japanese Society of Hypertension, JSH2000 Hypertension Guidelines for General Practitioners, 1st edition, The Japanese Society of Hypertension, June 30, 2000, page 55-58), hypocholesterolemic action of pratosartan reduces this hindrance.

Diuretics used are sulfonamide-, phenoxyacetic acid- and thiazide-type diuretics and others. Also, among these diuretics, especially sulfonamide- and thiazide-type diuretics have an adverse effect of increasing serum uric acid level, but co-administration of pratosartan reduces the increase of uric acid level which is an adverse effect of these diuretics.

The above sulfonamide-type diuretics are acetazolamide, metazolamide, ethoksuzolamide, clofenamide, dichlorfenamide, disulfamide, mefluside, chlorthalidone, kinetazone, furosemide, clopamide, tripamide, indapamide, crolexolone, metrazone, xipamide, bumetanide, piretanide and others. Also, the above thiazide-type diuretics are hydrochlorthiazide, methiclothiazide, benzylchlorthiazide, trichlormethiazide, cyclopenthiazide, polythiazide, ethiazide, cyclothiazide,bendroflumethiazide, hydroflumethiazide and others. Also, ethacrynic acid, thienilic acid, quincarbate, indaclinone and others. The other diuretics are triamterene, amiloride, spironolactone, potassium canrenoate and traxanox sodium. Suitable diuretics are thiazide-type diuretics, and hydrochlorthiazide is more suitable.

In this invention, in the case of co-administration of pratosartan and diuretics, the ratio of pratosartan and diuretics markedly varies, the ratio of mass is appropriate from 1:500 to 500:1.Both low dose of pratosartan and diuretics, which does not occur adverse effects, is able to lower blood pressure synergistically.

Also, co-administration of pratosartan and conventional hypolipidemic drugs is able to reinforce hypolipidemic action. Because pratosartan itself has hypotensive action, it is expected that the co-administartion of pratosartan and diuretics show a prevention and therapy for geriatric diseases such as hypertension and hyperlipidemia, and cardiovascular disorder induce by the duplication of the above-mentioned diseases. This conventional hypolipidemic drugs are at least one sort chosen from the group consist of statins, fibrates, cholesterol absorption inhibitors, cholesterol sequestrants and cholesterol excretion enhancers. The above statins are atorvastatin, simvastatin, pravastatin, fluvastatin, pitavastatin and others. Fibrates are clinofibrate, clofibrate, simfibrate, fenofibrate, bezafibrate and others. Cholesterol absorption inhibitors are ezetimibe, soysterol and others. Cholesterol sequestrants are cholestimide, cholestyramine and others. Cholesterol excretion enhancers are probucol and others. In the case of co-administration of pratosartan and conventional hypolipidemic drugs, the ratio of pratosartan and conventional hypolipidemic drugs markedly varies, the ratio of mass is appropriate from 1:500 to 500:1. Both low dose of pratosartan and conventional hypolipidemic drugs, which does not occur adverse effects, is able to lower serum lipid level, especially LDL level to the target value of therapy shown in The Guideline of Diagnosis and Treatment of Hyperlipidemia (Japan Atherosclerosis Society, 1997).

Working examples (experimental examples) are shown for the explanation of the invention in more detail as follows, but these examples do not limit this invention.

### EXAMPLE 1

The uricosuric action is estimated by the delayed disappearance of blood phenol red level as a marker. Namely, male 6 weeks old SD rats were orally administered 0.5 % methylcellulose (MC) or pratosartan (30 mg/kg or 100 mg/kg), and after three hours, phenol red solution (75 mg/kg) was intravenously administered. One hour after phenol red administration, blood sample was collected from abdominal aorta under ether anesthesia, and the amount of phenol red in serum was determined. The amount of phenol red level in control group was taken as 100%, and the delayed rate of phenol red disappearance from blood in pratosartan treated group was estimated as the enhance rate of uric acid excretion (%). In table 1 , experimental groups, subject substances, doses and the enhance rates of uric acid excretion (mean ± standard deviation). As shown table 1, pratosartan enhance the excretion of uric acid.

**Table 1**

| Groups | Subject substances and doses | The enhance rate of uric acid excretion (%) |
|---|---|---|
| Group1 | 0.5% MC 2mL/kg | 100±5 |
| Group2 | pratosartan 30mg/kg | 105±10 |
| Group3 | pratosartan 100mg/kg | 172±17 |

### EXAMPLE 2

Studies were carried out intended for mild and moderate essential hypertension. 17 patients complicated with hyperuricemia were administered pratosartan, and serum uric acid levels (mg/dL) before drug administration and the last day during administration were determined. The results were shown in table 2. Further, the dose of from 40 to 160 mg/day was administered every four weeks by degrees if the hypotensive action was insufficient.

**Table 2**

| No. of patients | Serum uric acid level (mg/dL) | | |
|---|---|---|---|
| | Before drug administration | The last day during administration | Difference |
| 17 | 8.0±1.1 | 7.4±1.0 | -0.7±1.3 |

### EXAMPLE 3

The systolic blood pressure level of male 20 weeks old SHR (Spontaneously hypertensive rat, SPF grade) was measured by tail cuff methods, and animals were randomized into 4 groups to balance the systolic blood pressure level among groups. 0.5 % methylcellulose (MC) or drugs suspended in 0.5 % MC were administered for 28 days, and systolic blood pressure levels after 5 hours of drug administration were measured at day 1, 7, 14 and 28. In table 3 , experimental groups, subject substances, doses, and the measured blood pressure levels (mean ± standard deviation) were shown in table 4. Further, the subject substances were hydrochlorothiazide (HCTZ), pratosartan, and co-administration of HCTZ and pratosartan. The dose of HCTZ and pratosartan were 10 mg/kg and 3 mg/kg, respectively, and the dosage volume was 2 mL/kg in each case. The hypotensive action synergistically enhanced that of HCTZ.

**Table 3**

| Groups | Subject substances and doses | |
|---|---|---|
| Group1 | 0.5% MC | 2mL/kg |
| Group2 | HCTZ | 10mg/kg |
| Group3 | Pratosartan | 3mg/kg |
| Group4 | HCTZ 10mg/kg+pratosartan | 3mg/kg |

**Table 4**

| | Group1 | Group2 | Group3 | Group4 |
|---|---|---|---|---|
| Before drug administration | 220±4 | 222±3 | 220±3 | 221±4 |
| Day 1 | 223±4 | 220±2 | 206±2 | 187±2 |
| Day 7 | 221±3 | 219±3 | 190±3 | 183±1 |
| Day 14 | 221±5 | 219±4 | 190±1 | 180±1 |
| Day 28 | 219±4 | 214±2 | 189±1 | 181±2 |

### EXAMPLE 4

Male 6 weeks old SD rats were fed chow containing 1 % cholesterol and 0.5 % cholic acid, and pratosartan at a dose of 0.3 mg/kg or 3 mg/kg were administered once a day for 8 weeks simultaneously. Twenty-four hours after the last dose, blood samples were collected from abdominal aorta under ether anesthesia, and serum total cholesterol level and LDL cholesterol levels were determined. The results were shown in figure 1 and 2. In figures, A is cholesterol value in the case of no pratosartan administration, B is cholesterol value in the case of pratosartan administration at a dose of 0.3 mg/kg and B is cholesterol value in the case of pratosartan administration at a dose of 3 mg/kg.

### EXAMPLE 5

107 patients with mild or moderate essential hypertension were administered at dose of from 40 to 160 mg/day was administered every four weeks by degrees if the hypotensive action was insufficient. Serum cholesterol level before pratosartan administration was compared with that of the last day during pratosartan administration in each patient. Also, a part of patients were other co-administered pratosartan and conventionally known hypolipidemic drugs. General names of co-administered drugs, doses and number of patients were as follows. Namely, the list was pravastatin (10 mg/day, 3 patients), simvastatin (5 mg/day, 3 patients), cerivastatin (0.15 mg/day, 2 patients), atorvastatin (5 mg/day, 1 patients), fenofibrate (150 mg/day, 1 patient) and probucol (500 mg/day, 1 patient). The results when the patients were classified in the presence and absence of other co-administered drugs, were shown in table 6 and 7. The result in the presence of co-administered drugs was shown in table 6, and the result in the absence of co-administered drug was shown in figure 7. Further, data were shown mean ± standard deviation.

**Table 5**

| No. of patients | Serum cholesterol level (mg/dL) | | | Paired t-test |
|---|---|---|---|---|
| | Before drug administration | The last day during administration | Difference | |
| 107 | 211.2±42.6 | 199.6±40.2 | -11.7±29.7 | P<0.0001 (significantly) |

**Table6**

| No. of patients | Serum cholesterol level (mg/dL) | | | Paired t-test |
|---|---|---|---|---|
| | Before drug administration | The last day during administration | Difference | |
| 12 | 250.25±45.16 | 214.58±37.31 | -35.67±41.49 | P<0.05 (significantly) |

**Table7**

| No. of patients | Serum cholesterol level (mg/dL) | | | Paired t-test |
|---|---|---|---|---|
| | Before drug administration | The last day during administration | Difference | |
| 95 | 206.28±39.81 | 197.66±40.33 | - 8.62±26.60 | P<0.01 (significantly) |

### FIELD OF INDUSTRIAL APPLICATION

Drugs in this invention are useful for treating or preventing duplication of hypertension with hyperuricemia and/or hypercholesterolemia which are much frequency among the overlap of the risk factor in geriatric diseases.

## Claims

1. Drugs for treating or preventing duplication of hypertension with serum hyperuricemia and/or hypercholesterolemia of which active principles are 2-propyl-3- {[2'-(1H-tetrazole -5-yl) biphenyl -4-yl]methyl}-5, 6, 7, 8-tetrahydrocyclohepta imidazole -4-(3H) - one and that prodrugs or that salts.

2. Drugs for treating or preventing duplication of hypertension with serum hyperuricemia of which active principles are 2-propyl-3- {[2'-(1H-tetrazole -5-yl) biphenyl -4-yl]methyl}-5, 6, 7, 8-tetrahydrocyclohepta imidazole -4-(3H) - one and that prodrugs or that salts with diuretics.

3. Drugs for treating or preventing duplication of hypertension with serum hyperuricemia of claim 2, wherein diuretics is one or more diuretics selected from the group consisting of sulfonamide-, phenoxyacetic acid-, thiazide-type diuretics, triamterene, amiloride, spironolactone, potassium canrenoate and traxanox sodium

4. Drugs for treating or preventing duplication of hypertension with serum hyperuricemia of claim 2, wherein diuretics is one or more thiazide-type diuretics selected from the group consisting of hydrochlorothiazide, methyclothiazide, benzylhydrochlorothiazide, trichloromethiazide, cyclopenthiazide, polythiazide, ethiazide; cyclothiazide, bendroflumethiazide and hydroflumethiazide.

5. Drugs for treating or preventing duplication of hypertension with hypercholesterolemia of which active principles are 2-propyl-3- {[2'-(1H-tetrazole -5-yl) biphenyl -4-yl]methyl}-5, 6, 7, 8-tetrahydrocyclohepta imidazole -4-(3H) - one and that prodrugs or that salts with hypolipidemic drug.

6. Drugs for treating or preventing duplication of hypertension with hypercholesterolemia of claim 5, wherein hypolipidemic drugs is one or more hypolipidemic diuretics selected from the group consisting of statins, fibrates, cholesterol absorption inhibitors, cholesterol sequestrants and cholesterol excretion enhancers.
